# EUROPÄISCHE PATENTSCHRIFT

(11) **EP 1 288 190 B1**
(45) Veröffentlichungstag und Bekanntmachung des Hinweises auf die Patenterteilung: **10.01.2007**
(21) Anmeldenummer: 02017778.8
(22) Anmeldetag: 12.08.2002
(51) Int. Cl.: C07C 209/78, C07C 263/10, C08G 18/76

(54) **Verfahren zur Herstellung von Polyisocyanaten der Diphenylmethanreihe mit vermindertem Farbwert**
Process for the preparation of polyisocyanates of the diphenylmethane series having reduced colour
Procédé de préparation de polyisocyanates de la série du diphénylméthane avec couleur atténuée

(30) Priorität: 24.08.2001 DE 10141620
(43) Veröffentlichungstag der Anmeldung: 05.03.2003
(73) Patentinhaber: Bayer MaterialScience AG, 51368 Leverkusen (DE)
(72) Erfinder: Hagen, Torsten, Dr., 40221 Düsseldorf (DE); Kämper, Friedhelm, Dr., 47807 Krefeld (DE); Koch, Daniel, Dr., 47137 Duisburg (DE); Müller, Heinz-Herbert, Dr., 47809 Krefeld (DE)

(56) Entgegenhaltungen:
- EP-A- 0 567 881
- DE-A- 19 804 915
- US-A- 6 031 136
- DATABASE CA [Online] CHEMICAL ABSTRACTS SERVICE, COLUMBUS, OHIO, US; retrieved from STN Database accession no. 104:68575/DN, HCAPLUS XP002237339 & JP 60 199861 A (MITSUI TOATSU CHEMICALS) 9. Oktober 1985 (1985-10-09)

## Beschreibung

Die Erfindung betrifft ein Verfahren zur Herstellung von Polyaminen der Diphenylmethanreihe sowie ein Verfahren zur Herstellung von Polyisocyanaten der Diphenylmethanreihe mit einem vermindertem Farbwert, welche durch Umsetzung der entsprechenden Polyamine der Diphenylmethanreihe mit Phosgen gewonnen werden.

Unter Polyisocyanaten der Diphenylmethanreihe werden Isocyanate und Isocyanatgemische folgenden Typs verstanden:

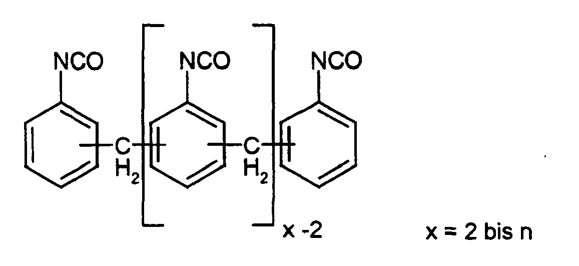

Analog werden unter Polyaminen der Diphenylmethanreihe Verbindungen und Verbindungsgemische folgenden Typs verstanden:

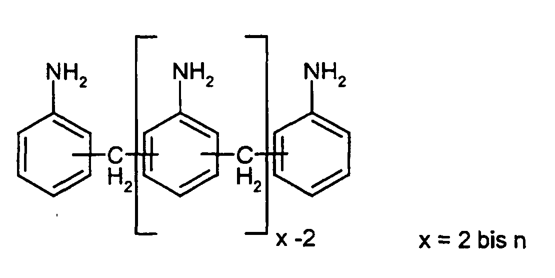

Die großtechnische Herstellung von Isocyanaten durch Umsetzung von Aminen mit Phosgen in Lösungsmitteln ist bekannt und in der Literatur ausführlich beschrieben (Ullmanns Enzyklopädie der technischen Chemie, 4. Auflage, Band 13, Seite 347-357, Verlag Chemie GmbH, Weinheim, 1977). Auf Basis dieses Verfahrens wird das Polyisocyanatgemisch hergestellt, das als Polyisoyanatkomponente bei der Herstellung von Polyurethanschäumen und anderen nach dem Polyadditionsverfahren hergestellten Polyurethankunststoffen dient.

Es ist allgemein bekannt, dass bei diesem Verfahren auch unerwünschte Farbstoffe oder farbgebende Komponenten gebildet werden, die auch bei der Weiterverarbeitung zu Polyurethanschäumen oder anderen Polyurethankunststoffen erhalten bleiben. Obwohl die Eigenfarbe der Polyisocyanat-Polyadditionsprodukte deren mechanische Eigenschaften nicht negativ beeinflusst, werden vom Verbraucher im wesentlichen farblose Produkte gewünscht. Als Maß für die Verfärbung des Polyisocyanates dient die Extinktion bei verschiedenen Wellenlängen.

Daher ist seit längerer Zeit die Reduzierung der Farbwerte von Polyisocyanaten der Diphenylmethanreihe das Ziel zahlreicher Versuche und Arbeiten, die in der Literatur beschrieben sind. So beschreibt beispielsweise DE-A1-4208359 die Behandlung von solchen Isocyanaten mit Wasserstoff in Gegenwart von Trägerkatalysatoren. DE-A1-4232769 beschreibt die Zugabe von Aminen, Harnstoffen und Antioxidantien zum Isocyanat. DE-A1-19815055 beschreibt die Farbverbesserung von Polyisocyanaten der Diphenylmethanreihe durch Bestrahlung mit Licht über einen längeren Zeitraum. DE-A1-19804915 beschreibt die Aufhellung von Polyisocyanaten der Diphenylmethanreihe durch eine komplizierte zeit- und temperaturgestufte Formaldehydzugabe auf der Polyaminstufe, die dann durch Phosgenierung in das gewünschte Isocyanat überführt wird.

EP-A-567 881 offenbart ein Verfahren zur Herstellung von Di- und Polyaminen der Diphenylmethanreihe, bei dem nach der Umsetzung von Anilin und Formaldehyd das Reaktionsgemisch neutralisiert und anschließend destillativ aufgearbeitet wird. EP-A-567 881 offenbart aber nicht, bei der Neutralisation die Base im Überschuss einzusetzen, um anschließend nach der Phosgenierung helle Di- und Polyisocyanate zu erhalten.

Nachteilig an allen diesen Vorgehensweisen ist, dass sie technisch aufwendig sind und/oder die Verwendung isocyanatfremder Hilfsstoffe erfordern oder wenig effizient sind.

Aufgabe der vorliegenden Erfindung war es daher, ein technisch einfaches und sicheres Verfahren bereit zu stellen, mit dem Polyisocyanate der Diphenylmethanreihe mit niedrigen Farbwerten hergestellt werden können. Es war ebenfalls Aufgabe der vorliegenden Erfindung, ein einfaches Verfahren zur Herstellung von Polyaminen der Diphenylmethanreihe bereit zu stellen, aus denen Polyisocyanate der Diphenylmethanreihe mit niedrigen Farbwerten durch Phosgenierung hergestellt werden können.

Die Aufgabe wird erfindungsgemäß gelöst durch ein Verfahren zur Herstellung von Polyaminen der Diphenylmethanreihe, bei dem man
a) Anilin und Formaldehyd in Gegenwart eines sauren Katalysators zu Polyaminen umsetzt und danach
b) das Reaktionsgemisch mit einer Base bei einer Temperatur von oberhalb 110°C neutralisiert oder das Reaktionsgemisch nach der Neutralisation mit einer Base auf eine Temperatur von oberhalb 110°C aufheizt, wobei man die Base in Mengen von ≥ 101% des für die Neutralisation des eingesetzten sauren katalysators stöchiometrisch benötigten Menge einsetzt.

Die Aufgabe wird ebenfalls erfmdungsgemäß gelöst durch ein Verfahren zur Herstellung von Polyisocyanaten der Diphenylmethanreihe, bei dem man
a) Anilin und Formaldehyd in Gegenwart eines sauren Katalysators zu Polyaminen umsetzt und danach
b) das Reaktionsgemisch mit einer Base bei einer Temperatur von oberhalb 110°C neutralisiert oder das Reaktionsgemisch nach der Neutralisation mit einer Base auf eine Temperatur von oberhalb 110°C aufheizt, wobei man die Base in Mengen von ≥ 101% des für die Neutralisation des eingesetzten sauren kalalysators stöchiometrisch benötigten Menge einsetzt, und danach.
c) die Polyamine durch Phosgenierung zu den entsprechenden Polyisocyanaten umsetzt.

Das erfindungsgemäße Verfahren kann sowohl kontinuierlich als auch diskontinuierlich durchgeführt werden.

Mit dem erfindungsgemäßen Verfahren können Polyisocyanate mit niedrigen Farbwerten hergestellt werden. Als Farbwert wird hierbei die gemessene Extinktion einer Lösung von Polyisocyanat in Monochlorbenzol, enthaltend 2 Gew.-% Polyisocyanat, bei einer Schichtdicke von 10 mm und Raumtemperatur gegen Monochlorbenzol bei definierten Wellenlängen verstanden.

Das im erfindungsgemäßen Verfahren eingesetzte Polyamin bzw. Polyamingemisch der Diphenylmethanreihe wird durch Kondensation von Anilin und Formaldehyd in Gegenwart eines sauren Katalysators erhalten (H.J. Twitchett, Chem. Soc. Rev. 3(2), 209 (1974), W. M. Moore in: Kirk-Othmer Encycl. Chem. Technol., 3^{rd}. Ed., New York, 2, 338-348 (1978)). Für das erfindungsgemäße Verfahren ist es dabei unerheblich, ob Anilin und Formaldehyd zunächst in Abwesenheit des sauren Katalysators vermischt werden und der saure Katalysator anschließend zugesetzt wird oder ob ein Gemisch aus Anilin und saurem Katalysator mit Formaldehyd zu Reaktion gebracht wird.

Geeignete Polyamingemische der Diphenylmethanreihe werden üblicherweise erhalten durch Kondensation von Anilin und Formaldehyd im Stoffmengenverhältnis 20 bis 1,6, bevorzugt 10 bis 1,8 sowie einem Stoffmengenverhältnis von Anilin und saurem Katalysator von 20 bis 1, bevorzugt 10 bis 2.

Formaldehyd wird technisch üblicherweise als wässrige Lösung eingesetzt. Es können jedoch auch andere methylengruppenliefernde Verbindungen wie z.B. Polyoxymethylenglykol, para-Formaldehyd oder Trioxan eingesetzt werden.

Als saure Katalysatoren haben sich starke organische und vorzugsweise anorganische Säuren bewährt. Geeignete Säuren sind beispielsweise Salzsäure, Schwefelsäure, Phosphorsäure, und Methansulfonsäure. Bevorzugt wird Salzsäure eingesetzt.

In einer bevorzugten Ausführungsform des Verfahrens wird Anilin und saurer Katalysator zunächst vereinigt. Dieses Gemisch wird, ggf. nach Abfuhr von Wärme, in einem weiteren Schritt mit Formaldehyd bei Temperaturen zwischen 20°C und 100°C, bevorzugt bei 30°C bis 70°C in geeigneter Weise vermischt und anschließend in einem geeigneten Verweilzeitapparat einer Vorreaktion zu unterzogen. Die Vorreaktion erfolgt bei Temperaturen zwischen 20°C bis 100°C, vorzugsweise im Temperaturbereich 30°C bis 80°C. Im Anschluss an Vermischung und Vorreaktion wird die Temperatur des Reaktionsgemisches in Stufen oder kontinuierlich und ggf. unter Überdruck auf eine Temperatur von 100°C bis 250°C, bevorzugt auf 100°C bis 180°C, besonders bevorzugt auf eine Temperatur von 100°C bis 160°C gebracht.

Es ist jedoch ebenfalls möglich in einer anderen Ausführungsform des Verfahrens Anilin und Formaldehyd zunächst in Abwesenheit des sauren Katalysators im Temperaturbereich von 5°C bis 130°C, bevorzugt von 40°C bis 100°C, besonders bevorzugt von 60°C bis 85°C, zu vermischen und so zur Reaktion zu bringen. Hierbei bilden sich Kondensationsprodukte aus Anilin und Formaldehyd (sog. Aminal). Im Anschluss an die Aminalbildung kann im Reaktionsgemisch vorhandenes Wasser durch Phasentrennung oder andere geeignete Verfahrensschritte, beispielsweise durch Destillation, entfernt werden. Das Kondensationsprodukt wird dann in einem weiteren Verfahrensschritt mit dem sauren Katalysator in geeigneter Weise vermischt und in einem Verweilzeitapparat bei 20°C bis 100°C, bevorzugt 30°C bis 80°C einer Vorreaktion unterzogen. Anschließend wird die Temperatur des Reaktionsgemisches in Stufen oder kontinuierlich und ggf. unter Überdruck auf eine Temperatur von 100°C bis 250°C, bevorzugt auf 100°C bis 180°C, besonders bevorzugt auf eine Temperatur von 100°C bis 160°C gebracht.

Die Umsetzung von Anilin und Formaldehyd in Gegenwart eines sauren Katalysators zu Polyaminen der Diphenylmethanreihe kann in Anwesenheit weiterer Stoffe (z.B. Lösungsmittel, Salze, organische und anorganische Säuren) geschehen.

Zur Aufarbeitung des sauren Reaktionsgemisches wird das Reaktionsgemisch mit einer Base neutralisiert. Nach dem Stand der Technik erfolgt die Neutralisation üblicherweise bei Temperaturen von beispielsweise 90 - 100°C (H.J. Twitchett, Chem. Soc. Rev. 3(2), 223 (1974)). Als Basen sind beispielsweise geeignet die Hydroxide der Alkali- und Erdalkalielemente. Vorzugsweise kommt wässrige NaOH zur Anwendung.

Im erfindungsgemäßen Verfahren wird die Neutralisation des sauren Reaktionsgemisches bei Temperaturen von oberhalb 110°C, bevorzugt im Bereich 111°C bis 300°C, besonders bevorzugt bei 115°C bis 200°C, ganz besonders bevorzugt 120°C bis 180°C, insbesondere ganz besonders bevorzugt 130 bis 160°C, durchgeführt. Alternativ wird die Neutralisation bei Temperaturen von unterhalb 110°C durchgeführt und das neutralisierte Reaktionsgemisch anschließend auf eine Temperatur von oberhalb 110°C, bevorzugt 111°C bis 300°C, besonders bevorzugt 115°C bis 200°C, ganz besonders bevorzugt 120°C bis 180°C, insbesondere ganz besonders bevorzugt 130 bis 160°C, aufgeheizt.

Die Neutralisation erfolgt beispielsweise in der Weise, dass das saure Reaktionsgemisch der Anilin/Formaldehydkondensation mit der Base vermischt und einem Verweilzeitapparat (beispielsweise Rührkessel, Rührkesselkaskade, Strömungsrohr, Umpumpreaktor) zugeführt wird. Bei geeigneten Verweilzeitapparaten (z.B. Rührkessel) kann die Vermischung von saurem Kondensationsgemisch und Base auch direkt im Verweilzeitapparat erfolgen.

Das neutralisierte Reaktionsgemisch wird vorzugsweise für eine Verweilzeit von ≥0,1 min, bevorzugt 0,1 bis 180 min, besonders bevorzugt 2 bis 120 min, ganz besonders bevorzugt 10 bis 60 min, auf einer Temperatur oberhalb von 110°C gehalten.

Zur Einstellung einer Temperatur gemäß der vorliegenden Erfindung kann Zufuhr oder Abfuhr von Wärme notwendig sein. Dies richtet sich insbesondere nach der gewünschten Temperatur, bei welcher die Neutralisation des erfindungsgemäßen Verfahrens erfolgen soll, aber auch nach der freiwerdenden Neutralisationswärme, der Temperatur der sauren Kondensationsmischung und der Temperatur der eingesetzten Base bzw. Basenlösung. Um das Sieden unterhalb der gewünschten Neutralisationstemperatur zu verhindern, muss das Verfahren gegebenenfalls bei erhöhtem Druck durchgeführt werden.

Die zur Neutralisation eingesetzte Base wird in Mengen von größer 100%, bevorzugt 101 bis 140%, vorzugsweise 105 bis 120% der für die Neutralisation des eingesetzten sauren Katalysators stöchiometrisch benötigten Menge eingesetzt. Der Effekt der Neutralisation unter erhöhter Temperatur auf die Farbe der Polyisocyanate der Diphenylmethanreihe wird verstärkt, wenn in dem Neutralisationsverweilzeitbehälter für eine ausreichende Durchmischung der organischen und wässrigen Phase gesorgt wird. Dies kann durch Anwendung der in der Technik bekannten Methoden beispielsweise durch statische oder dynamische Mischer oder Erzeugung von Turbulenz erfolgen.

Im Anschluss an die Neutralisation wird üblicherweise die organische von der wässrigen Phase durch geeignete Verfahren (z.B. Phasentrennung in einer Scheideflasche) getrennt. Diese Trennung von organischer und wässriger Phase kann bei der selben Temperatur erfolgen, bei der die Neutralisation des sauren Umlagerungsgemisches erfolgte. Die nach Abtrennung der wässrigen Phase verbleibende produktenthaltende organische Phase wird üblicherweise weiteren Aufarbeitungsschritten unterzogen (z.B. Wäsche) und anschließend von überschüssigem Anilin und anderen im Gemisch vorhandenen Stoffen (z.B. weiteren Lösungsmitteln) durch geeignete physikalische Trennverfahren wie z.B. Destillation, Extraktion oder Kristallisation befreit.

Das so erhaltene Polyamin der Diphenylmethanreihe (Roh-MDA) wird nach den bekannten Methoden mit Phosgen in einem inerten organischen Lösungsmittel zu den entsprechenden Isocyanaten umgesetzt. Das Molverhältnis von Roh-MDA zu Phosgen wird zweckmäßigerweise so bemessen, dass pro Mol NH₂-Gruppe 1 bis 10 Mol, vorzugsweise 1,3 bis 4 Mol Phosgen in der Reaktionsmischung vorliegen. Als inerte Lösungsmittel haben sich chlorierte, aromatische Kohlenwasserstoffe wie z.B. Monochlorbenzol, Dichlorbenzole, Trichlorbenzole, die entsprechenden Toluole und Xylole sowie Chlorethylbenzol bewährt. Insbesondere Anwendung finden als inerte organische Lösungsmittel Monochlorbenzol, Dichlorbenzol oder Mischungen dieser Chlorbenzole. Die Menge an Lösungsmittel wird vorzugsweise so bemessen, dass die Reaktionsmischung einen Isocyanatgehalt von 2 bis 40 Gew.-%, vorzugsweise zwischen 5 und 20 Gew.-%, bezogen auf das Gesamtgewicht der Reaktionsmischung, aufweist. Nach beendeter Phosgenierung werden aus der Reaktionsmischung das überschüssige Phosgen, das inerte organische Lösungsmittel, die gebildete HCl oder Mischungen davon durch Destillation abgetrennt.

Das nach dem erfindungsgemäßen Verfahren hergestellte Roh-MDI besitzt eine deutlich reduzierte Färbung. Es lassen sich jedoch noch weitere analytische Unterschiede im hergestellten MDI nachweisen (wie z.B. ein erhöhter Gehalt an Isocyanatgruppen).

### Beispiele:

### Beispiel 1 (Vergleichsbeispiel)

Zu 410 g Anilin wurden bei 80°C innerhalb von 20 min zugleich 707,6 g Anilin und 563,6 g einer 32,0%igen wässrigen Formaldehydlösung getropft. Nach der Zugabe wurde noch 10 min gerührt und anschließend eine Phasentrennung bei 70-80°C vorgenommen. Von der organischen Phase wurde eine Menge von 284,5 g auf 35°C temperiert und anschließend bei dieser Temperatur innerhalb von 29 min mit der restlichen organischen Phase und 341,9 g einer 32,0%igen wässrigen Salzsäure versetzt. Nach beendeter Zugabe und einer 30 minütigen Nachrührzeit bei dieser Temperatur wurde während 10 min auf 60°C aufgeheizt und 30 min bei dieser Temperatur gehalten. Anschließend wurde innerhalb von 30 min auf Rückflusstemperatur aufgeheizt und 10 h unter Rückfluss gerührt. Zu 815 g des so erhaltenen sauren Umlagerungsgemisches wurden 154,3 g einer 49,6%igen wässrigen Natronlauge und 213 ml siedendes Wasser gegeben. Nach 15 min Rühren unter Rückfluss wurde bei 80-90°C eine Phasentrennung vorgenommen und die organische Phase weitere 2x mit je 638 ml siedendem Wasser gewaschen. Die organische Phase wurde anschließend unter vermindertem Druck vom überschüssigen Anilin befreit. Von dem so erhaltenen Polyamin wurden 50 g in 255 ml Chlorbenzol gelöst, auf 55°C erwärmt und innerhalb von 10 s unter intensivem Rühren zu einer auf 0°C temperierten Lösung von 105 g Phosgen in 310 ml Chlorbenzol gegeben. Die Suspension wurde unter Durchleiten von Phosgen innerhalb von 45 min auf 100°C und anschließend während 10 min auf Rückflusstemperatur aufgeheizt. Nach weiteren 10 min bei dieser Temperatur wurde das Lösungsmittel unter vermindertem Druck bis zu einer Sumpftempratur von 100°C abdestilliert. Das rohe Isocyanat wurde anschließend in einer Destillationsapparatur bei einem Druck von 4-6 mbar durch ein auf 260°C erwärmtes Heizbad bis zum ersten Produktübergang erhitzt und daraufhin innerhalb von 5 min auf Raumtemperatur abgekühlt. Von dem so erhaltenen Isocyanat wurde 1,0 g in Chlorbenzol gelöst und mit Chlorbenzol zu 50 ml verdünnt. Die so erhaltene Lösung besaß bei einer Wellenlänge von 430 nm, einer Schichtdicke von 10 mm und Raumtemperatur eine Extinktion von 0,193 gegen Chlorbenzol.

### Beispiel 2 (erfindungsgemäß)

699 g des obigen sauren Umlagerungsgemisches wurden mit 132,4 g 49,6%iger wässriger Natronlauge unter Zusatz von 60,8 g Wasser neutralisiert. Die Reaktionsmischung wurde in einen Druckbehälter überführt und 3 h bei 160°C und Eigendruck gerührt. Anschließend wurden die Phasen getrennt und die organische Phase analog Beispiel 1 gereinigt. Das resultierende Polyamin wurde analog Beispiel 1 mit Phosgen in das entsprechende Isocyanat überführt. Die Extinktion bei 430 nm gemäß der in Beispiel 1 beschriebenen Methode betrug 0,163.

### Beispiel 3 (erfindungsgemäß)

403 g eines sauren Umlagerungsgemisches, dessen Herstellung analog Beispiel 1 erfolgte, wurden mit 76 g 50%iger wässriger Natronlauge unter Zusatz von 105 g Wasser neutralisiert. Die Reaktionsmischung wurde in einen Druckbehälter überführt und 15 min bei 160°C und Eigendruck gerührt. Anschließend wurden die Phasen getrennt und die organische Phase analog Beispiel 1 gereinigt. Das resultierende Polyamin wurde analog Beispiel 1 mit Phosgen in das entsprechende Isocyanat überführt. Die Extinktion bei 430 nm gemäß der in Beispiel 1 beschriebenen Methode betrug 0,164.

## Patentansprüche

1. Verfahren zur Herstellung von Polyaminen der Diphenylmethanreihe, bei dem man
a) Anilin und Formaldehyd in Gegenwart eines sauren Katalysators zu Polyaminen umsetzt und danach
b) Das Reaktionsgemisch mit einer Base bei einer Temperatur von oberhalb 110°C neutralisiert oder das Reaktionsgemisch nach der Neutralisation mit einer Base auf eine Temperatur von oberhalb 110°C aufheizt, wobei man die Base in Mengen von ≧ 101 % der für die Neutralisation des eingesetzten sauren Katalysators stöchiometrisch benötigten Menge eingesetzt.

2. Verfahren nach Anspruch 1, bei dem man die Neutralisation mit einer Base auf eine Temperatur von 120°C bis 180°C durchführt.

3. Verfahren nach Anspruch 1 oder 2, bei dem man als Base wässrige NaOH einsetzt.

4. Verfahren zur Herstellung Polyisocyanaten der Diphenylmethanreihe, bei dem man die Polyamine der Diphenylmethanreihe nach dem Verfahren nach Anspruch 1 herstellt, und die Polyamine durch Phosgenierung zu den entsprechenden Polyisocyanaten umsetzt.

5. Verfahren nach Anspruch 4, bei dem man die Neutralisation bei einer Temperatur von 120° C bis 180°C durchführt.

6. Verfahren nach Anspruch 4 oder 5, bei dem man als Base wässrige NaOH einsetzt.

## Claims

1. Process for the production of polyamines of the diphenylmethane series, in which
a) aniline and formaldehyde are reacted in the presence of an acidic catalyst to give polyamines and then
b) the reaction mixture is neutralised with a base at a temperature of more than 110°C or the reaction mixture is heated to a temperature of more than 110°C after neutralisation with a base, the base being used in quantities of ≥ 101% of the quantity required stoichiometrically for the neutralisation of the acidic catalyst used.

2. Process according to claim 1, in which the neutralisation with a base is performed at a temperature of 120°C to 180°C.

3. Process according to claim 1 or 2, in which aqueous NaOH is used as the base.

4. Process for the production polyisocyanates of the diphenylmethane series, in which the polyamines of the diphenylmethane series are produced by the process according to claim 1 and the polyamines are converted to the corresponding polyisocyanates by phosgenation.

5. Process according to claim 4, in which the neutralisation is performed at a temperature of 120°C to 180°C.

6. Process according to claim 4 or 5, in which aqueous NaOH is used as the base.

## Revendications

1. Procédé pour la préparation de polyamines de la série du diphénylméthane dans lequel :
a) on fait réagir l'aniline et le formaldéhyde en présence d'un catalyseur acide avec formation d'une polyamine, puis
b) on neutralise le mélange de réaction à l'aide d'une base à une température supérieure à 110°C ou bien on chauffe le mélange de réaction à une température supérieure à 110°C après neutralisation à l'aide d'une base, la base étant mise en oeuvre en quantité supérieure ou égale à 101 % de la quantité théorique nécessaire pour la neutralisation du catalyseur acide mis en oeuvre.

2. Procédé selon la revendication 1, dans lequel la neutralisation est réalisée à l'aide d'une base à une température de 120 à 180°C.

3. Procédé selon la revendication 1 ou 2, dans lequel la base utilisée est une solution aqueuse de NaOH.

4. Procédé pour la préparation de polyisocyanates de la série du diphénylméthane dans lequel on prépare les polyamines de la série du diphénylméthane par le procédé selon la revendication 1 et on convertit ces polyamines par phosgénation en les polyisocyanates correspondants.

5. Procédé selon la revendication 4, dans lequel on procède à la neutralisation à une température de 120 à 180°C.

6. Procédé selon la revendication 4 ou 5, dans lequel la base utilisée est une solution aqueuse de NaOH.
